(19) Europäisches Patentamt
European Patent Office
Office européen des brevets

(11) **EP 2 939 667 A1**

(12) # EUROPEAN PATENT APPLICATION
published in accordance with Art. 153(4) EPC

(43) Date of publication:
**04.11.2015 Bulletin 2015/45**

(21) Application number: **13867096.3**

(22) Date of filing: **26.12.2013**

(51) Int Cl.:
*A61K 9/70* (2006.01)   *A61J 1/00* (2006.01)
*A61K 31/27* (2006.01)   *A61K 31/465* (2006.01)
*A61K 47/32* (2006.01)   *A61M 37/00* (2006.01)
*A61P 25/28* (2006.01)   *A61P 25/34* (2006.01)

(86) International application number:
**PCT/JP2013/084950**

(87) International publication number:
**WO 2014/104220 (03.07.2014 Gazette 2014/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.12.2012 JP 2012288950
19.08.2013 JP 2013169987**

(71) Applicant: **NIPRO Patch Co., Ltd.
Saitama 344-0057 (JP)**

(72) Inventors:
• **KAWAMURA, Naohisa
Kasukabe-shi
Saitama 344-0057 (JP)**
• **SAWADA, Hidenori
Kasukabe-shi
Saitama 344-0057 (JP)**

(74) Representative: **Ström & Gulliksson AB
P.O. Box 4188
203 13 Malmö (SE)**

(54) **PACKAGING**

(57)    The objective of the present invention is to provide a method of preventing an oxygen-caused alteration of a drug in an adhesive patch enclosed inside a packaging container. The present invention is a package body comprising a packaging container and a drug-containing adhesive patch enclosed inside the packaging container, wherein the amount of oxygen gas inside the package body is 20 μL or less, and the concentration of oxygen gas is 17.0 to 25.0 vol%. This packaging does not have to contain an oxygen scavenger as a separate member distinct from the drug-containing adhesive patch. Further, the amount of oxygen gas may be 4 μL/mg or less per amount of a drug in the drug-containing adhesive patch.

FIG. 1A

EP 2 939 667 A1

**Description**

TECHNICAL FIELD

The present invention relates to a package body.

BACKGROUND ART

An adhesive patch is a formulation generally having a drug-containing adhesive layer, and is attached on the skin and the like when used. An adhesive patch is stored in a sealed packaging container in order to prevent an alteration and the like of a drug in the adhesive patch. Various factors may cause an alteration and the like of a drug in an adhesive patch, including, for example, oxygen, light and the like.

As a method of preventing an alteration of a drug in an adhesive patch due to oxygen, the followings are used: a method in which an anti-oxidant is blended into an adhesive patch; a method in which an oxygen scavenger and the like is enclosed inside a packaging container along with an adhesive patch; and the like. However, these methods cost time and expense for blending an anti-oxidant or enclosing an oxygen scavenger and the like. Therefore, a more convenient method has been demanded.

As another method of preventing an alteration of a drug in an adhesive patch due to oxygen, proposed is a method in which the concentration of oxygen gas inside a packaging container is limited to 3.0 vol% or less (Patent Document 1). Further, among drugs which can be formulated into an adhesive patch, rivastigmine is known as an agent for suppressing symptom progression of Alzheimer type dementia. Rivastigmine-containing formulations are prepared in various dosage forms such as a rivastigmine-containing adhesive patch. For example, a rivastigmine-containing adhesive patch is commercially available under the product name "Exelon®".

Rivastigmine contained in a rivastigmine-containing adhesive patch may be altered due to oxygen, light and the like during storage of the adhesive patch. Therefore, methods of storing an adhesive patch have been studied in which an alteration of rivastigmine is prevented. Such storing methods include, for example, a method in which a rivastigmine-containing adhesive patch is enclosed inside a packaging container composed of a multilayered film comprising a polyacrylonitrile layer and an aluminum layer (Patent Document 2).

Patent Document 1: Japanese Unexamined Patent Application, Publication No. 2012-51875
Patent Document 2: Japanese Patent No. 5093545

DISCLOSURE OF THE INVENTION

Problems to be Solved by the Invention

However, even the method described in Patent Document 1 wherein the concentration of oxygen gas inside a packaging container is limited to 3.0 vol% or less costs time and expense. Therefore a more convenient method has been demanded. Further, Patent Document 1 describes a method of limiting the concentration of oxygen gas inside a packaging container to 3.0 vol% or less, the method comprising mainly enclosing an oxygen scavenger along with an adhesive patch. However, an oxygen scavenger is often bulky, resulting in an increased intercontainer space.

Moreover, there may be an occasion where the packaging container described in Patent Document 2 can not sufficiently control an alteration of rivastigmine. Therefore, a method of storing a rivastigmine-containing adhesive patch has been demanded in which an alteration of rivastigmine is more reliably prevented.

The present invention is made in order to solve the above problems. An object of the present invention is to provide a method of preventing an oxygen-caused alteration of a drug in an adhesive patch enclosed inside a packaging container.

Further, another object of the present invention is to provide a method of preventing an alteration of rivastigmine in a rivastigmine-containing adhesive patch enclosed inside a packaging container.

Means for Solving the Problems

The present inventors find that the above problems can be solved by limiting the amount and concentration of oxygen gas inside a packaging container within predetermined ranges. Then the present invention has been completed.

Further, the present inventors find that the above problems can be solved by using a packaging container composed of a multilayered film comprising a predetermined layer as a storage container for a rivastigmine-containing adhesive patch. Then the present invention has been completed.

Specifically, the present invention provides the followings.

(1) A package body comprising a packaging container and a drug-containing adhesive patch enclosed inside the packaging container,
wherein the amount of oxygen gas inside the package body is 20 μL or less, and the concentration of oxygen gas is 17.0 to 25.0 vol%.
(2) The package body according to (1), wherein the package body does not contain an oxygen scavenger as a separate member distinct from the drug-containing adhesive patch.
(3) The package body according to (1) or (2), wherein the amount of oxygen gas is 4 μL/mg or less per amount of a drug in the drug-containing adhesive patch.
(4) The package body according to any one of (1) to (3), wherein the drug is any of nicotine, rivastigmine or pharmacologically acceptable salts thereof.
(5) The package body according to any one of (1) to (4), wherein the drug-containing adhesive patch comprises a support and an adhesive layer disposed on the support, the adhesive layer comprising an acrylic adhesive substantially free from a carboxyl group.
(6) A package body comprising a packaging container and an adhesive patch enclosed inside the packaging container, wherein the adhesive patch comprises a support and an adhesive layer containing a drug and disposed on the support, the drug comprises at least rivastigmine and/or a pharmaceutically acceptable salt thereof, and
the packaging container comprises a multilayered film in which at least a polyacrylonitrile layer, an aluminum layer and a polyethylene terephthalate layer are laminated.
(7) The package body according to (6), wherein the innermost layer of the packaging container is a polyacrylonitrile layer.
(8) The package body according to (7), wherein the multilayered film is a multilayered film in which a polyacrylonitrile layer, an aluminum layer and a polyethylene terephthalate layer are laminated in this order.
(9) The package body according to any one of (6) to (8), wherein the polyacrylonitrile layer has a thickness of 10 to 50 μm, and the aluminum layer has a thickness of 1 to 15 μm, and the polyethylene terephthalate layer has a thickness of 5 to 20 μm.
(10) The package body according to any one of (6) to (9), wherein the package body does not contain any one or more of an oxygen scavenger and an anti-oxidant.
(11) The package body according to any one of (6) to (10), wherein the adhesive layer comprises an acrylic adhesive.

Effects of the Invention

The present invention provides a method of preventing an oxygen-caused alteration of a drug in an adhesive patch enclosed inside a packaging container.
Further, the present invention provides a method of preventing an alteration of rivastigmine in a rivastigmine-containing adhesive patch enclosed inside a packaging container.

BRIEF DESCRIPTION OF THE DRAWINGS

Fig. 1A shows the content of a nicotine analog in adhesive patches enclosed in package bodies used in Example.
Fig. 1B shows the content of a nicotine analog in adhesive patches enclosed in package bodies used in Example.
Fig. 2 shows the change in color tone of adhesive patches enclosed in package bodies used in Example.
Fig. 3A shows the content of a rivastigmine analog in adhesive patches enclosed in package bodies used in Example.
Fig. 3B shows the content of a rivastigmine analog in adhesive patches enclosed in package bodies used in Example.
Fig. 4 shows the amounts and concentrations of oxygen gas in the package bodies according to one aspect of the present invention and the conventional package bodies.
Fig. 5 shows the amounts and concentrations of oxygen gas in the package bodies according to one aspect of the present invention and the conventional package bodies.

PREFERRED MODE FOR CARRYING OUT THE INVENTION

Below, embodiments of the present invention will be described. Note that the present invention shall not be limited to the following embodiments.

Package body - I

A first embodiment of the present invention provides a package body (in other words, a packaging product) comprising a packaging container and a drug-containing adhesive patch enclosed in the above packaging container, wherein the amount of oxygen gas in the above package body is 20 μL or less, and the concentration of oxygen gas is 17.0 to 25.0 vol%. Note that the term "package body" used in the present invention refers to a packaging container in a state where a drug-containing adhesive patch is enclosed inside. Further, a "drug-containing adhesive patch" may simply be referred to as an "adhesive patch." Moreover, a "packaging container" used in the present invention is intended for enclosing an adhesive patch, and may also be called a "packaging bag." Each component of a first package body according to the present invention will be described below.

(The amount and concentration of oxygen gas)

In the present invention, the amount of oxygen gas inside a package body is adjusted to 20 μL or less, and the concentration of oxygen gas is adjusted to 17.0 to 25.0 vol%. After conducting extensive studies, the present inventors find that the stability of a drug in an adhesive patch enclosed inside a packaging container is less deteriorated when the amount and concentration of oxygen gas fall within such ranges.

Conventionally, an oxygen scavenger, an anti-oxygen agent and the like are enclosed in order to reduce the amount of oxygen gas inside a package body. However, these agents merely reduce the amount of oxygen gas gradually during storage of an adhesive patch. Therefore, the stability of a drug in an adhesive patch is likely to be deteriorated before the amount of oxygen gas inside a package body is sufficiently reduced.

Further, for example, Japanese Unexamined Patent Application No. 2012-51875 describes a method in which the concentration of oxygen gas inside a packaging container is reduced to 3.0 vol% or less by means of decompression and the like. However, decompression alone results in a concentration of oxygen gas inside a package body close to the ratio of oxygen in the air (about 20 vol%). Therefore, additional use of an oxygen scavenger and the like is essential in order to reduce the amount of oxygen gas to 3.0 vol% or less in a package body. That is, decompression and the like described in Japanese Unexamined Patent Application No. 2012-51875 is merely an auxiliary means for efficiently reducing the amount of oxygen gas inside a package body to 3.0 vol% or less when a less amount of an oxygen scavenger is used. Therefore, an object thereof can not be considered to sufficiently reduce the total amount of gas inside a package body including oxygen gas as in the present invention.

Further, as another method for reducing the amount of oxygen gas inside a package body, substitution with inert gas is performed such as nitrogen substitution and argon substitution. However, when a specific element is substituted with oxygen, the total amount of the air inside a packaging container is the same as or higher than that before substitution although the concentration of oxygen gas inside a packaging container becomes low. Therefore, the volume of the resulting package body increases (in this case, the concentration of oxygen gas inside the package body is likely to be about 1.0 to 5.0 vol%). As a result, an adhesive patch may be twisted or bent in a package body, resulting in a poor storagability of the adhesive patch. Further, substitution with inert gas costs time and expense in a manufacturing process.

In contrast, since the amount of oxygen gas is as small as 20 μL or less at a stage where an adhesive patch is enclosed inside the packaging container according to the present invention, the stability of a drug is less deteriorated in the adhesive patch during storage of the adhesive patch. Further, the concentration of oxygen gas inside a packaging container is 17.0 to 25.0 vol%, and the total gas content inside the packaging container is small, preventing an increased volume of a package body.

The amount of oxygen gas inside a package body is 20 μL or less, preferably 15 μL or less, more preferably 10 μL or less in order to reduce the effects of oxygen on a drug in an adhesive patch. Further, although the amount of oxygen gas inside a package body is preferably zero, an acceptable amount is 0.01 μL or more, preferably 0.1 μL or more since complete removal is difficult. The amount of oxygen gas inside a package body may be 4 μL/mg or less per amount of a drug in an adhesive patch, preferably 0.002 to 3 μL/mg in order to reduce the effects of oxygen on the drug in the adhesive patch.

The concentration of oxygen gas inside a package body may be a concentration close to the ratio of oxygen in the air (about 20 vol%), and is 17.0 to 25.0 vol%, preferably 18.0 to 24.0 vol%.

As a method of adjusting the amount and concentration of oxygen gas inside a package body to the aforementioned ranges, those in which the amount of gas inside a package body is physically reduced can be used. Such methods include those in which decompression or pressing is used, specifically, those in which a vacuum pump, a compressor and the like are used. According to these methods, the total gas content inside a packaging container can be reduced while the concentration of oxygen gas inside the packaging container is maintained to be close to the ratio of oxygen in the air (about 20 vol%), achieving the aforementioned amount and concentration of oxygen gas.

In the present invention, methods such as nitrogen substitution and argon substitution are preferably not used in order to set the concentration of oxygen gas inside a package body to the aforementioned range. The reason for this is that the concentration of oxygen gas inside a package body is difficult to be set within the aforementioned range because these methods increase the ratio of a specific element inside a package body.

The total gas content inside the package body according to the present invention may be 0.5 to 180 μL, preferably 5.0 to 120 μL, more preferably 10 to 100 μL. The package body according to the present invention has a smaller total gas content than the conventional package body.

The package body according to the present invention can be obtained by placing an adhesive patch inside a packaging container, and adjusting the amount and concentration of oxygen gas inside the packaging container to the aforementioned ranges, and then sealing the packaging container by a method such as heat sealing, sealing with an adhesive and the like. The package body according to the present invention has a sufficiently reduced amount and concentration of oxygen gas inside a package body without an oxygen scavenger enclosed, and therefore, preferably does not contain an oxygen scavenger as a separate member distinct from an adhesive patch. The package body according to the present invention may consist of a packaging container and a drug-containing adhesive patch enclosed in the above packaging container.

The concentration of oxygen gas inside a packaging container can be determined with an oxygen-gas concentration meter. The amount of oxygen gas inside a packaging container can be determined based on the total gas content and the concentration of oxygen gas inside a package body.

(Packaging container)

There is no particular limitation for the packaging container as long as it can be hermetically sealed, and those commonly used as packaging containers for adhesive patches such as tapes and cataplasms.

(Drug-containing adhesive patch)

There is no particular limitation for the form of an adhesive patch enclosed inside the packaging container according to the present invention, and known adhesive patch having a drug-containing layer can be used. For example, it may be an adhesive patch comprising a drug-containing adhesive layer (a sheet-like formulation and the like), or an adhesive patch comprising a support and an adhesive layer (which usually contains a drug) disposed on the above support. Among these, preferred is an adhesive patch comprising a support and an adhesive layer disposed on the above support. Further, the adhesive patch may be those in which a known skin adhesive layer (polyisobutylene and the like), controlled-release membrane and strippable liner (an oxygen impermeable liner and the like) are laminated on an adhesive layer.

There is no particular limitation for the adhesive included in an adhesive layer of an adhesive patch as long as it is commonly used for adhesive patches to be applied to the skin, and it may be one or more of acrylic adhesives, rubber-based adhesives or a silicon-based adhesives.

Among the above adhesives, acrylic adhesives are preferred in view of that strength and the like are not lost even under a low-pressure environment where the total gas content inside a package body is small. Among acrylic adhesives, those substantially free from a carboxyl group are preferred in view of that they are less reactive with a drug, and can control the decrease in transdermal absorption of a drug, and are less affected by oxygen. The phrase "substantially free from a carboxyl group" means not only those not containing a carboxyl group at all but also those in which carboxyl groups are all theoretically replaced with substituents such as ester linkages. Those include, for example, a case in which a trace amount of ester linkages and the like are converted into free carboxyl groups due to hydrolysis, and a case in which free carboxyl groups are included as impurities from a raw material.

There is no particular limitation for the acrylic adhesive substantially free from a carboxyl group, and, for example, adhesives described in Japanese Unexamined Patent Application No. 2005-325101, Japanese Patent No. 3809462 and the like can be used. Among these, in particular, the acrylic adhesive described in Japanese Unexamined Patent Application No. 2005-325101 can be conveniently used.

An acrylic adhesive substantially free from a carboxyl group and a hydroxy group is particularly preferred as the acrylic adhesive in the present invention in view of that it is less reactive with a drug, and can control the decrease in transdermal absorption of the drug, and is less affected by oxygen.

There is no particular limitation for the drug contained in an adhesive patch, and even those more susceptive to alteration due to oxygen can be preferably used. Such drugs include any of nicotine (an smoking-cessation aid), rivastigmine (an therapeutic agent for Alzheimer-type dementia) or pharmacologically acceptable salts thereof. The amount of a drug in an adhesive patch may be appropriately adjusted according to the desired pharmacological effect.

Other components may be contained in an adhesive patch, if desired. Such components include adhesiveness-conferring agents (rosin ester and the like), plasticizing agents, antiseptic agents, pH adjusters, chelating agents, transdermal absorption promoting agents, excipients, flavoring agents, coloring agents, fatty acids, fats and oils and the like. Note that the stability of a drug in an adhesive patch is excellent in the package body according to the present invention even in a case where an anti-oxidant is not blended in the adhesive patch. However, an anti-oxidant such as dibutylhydroxytoluene may be blended into an adhesive patch, if desired.

(Method of manufacturing adhesive patch)

There is no particular limitation for the method of preparing an adhesive patch, and known preparation methods can be used for preparing an adhesive patch. They include the followings: for example, a method of obtaining an adhesive patch, comprising uniformly dissolving each component into a solution, and coat-drying the resulting solution using a coater; a method of obtaining an adhesive patch, comprising uniformly stirring components of an adhesive layer, and coat-drying the resulting composition onto a surface of a support using a coater.

Package body - II

In a second embodiment of the present invention, provided is a package body comprising a packaging container and an adhesive patch enclosed in the above packaging container. Note that the term "package body" used in the present invention refers to a packaging container in a state where an adhesive patch is enclosed inside. Further, an "adhesive patch" used in the present invention is also referred to as a "rivastigmine-containing adhesive patch." Moreover, a "packaging container" used in the present invention is intended for enclosing an adhesive patch therein, and may also be called a "packaging bag." Below, each component of the second package body according to the present invention will be described.

(Configuration of packaging container)

The packaging container according to the present invention comprises a multilayered film in which at least a polyacrylonitrile layer (hereinafter, the polyacrylonitrile layer is also referred to as "PAN"), an aluminum layer (hereinafter, the aluminum layer is also referred to as "AL") and a polyethylene terephthalate layer (hereinafter, the polyethylene terephthalate layer is also referred to as "PET") are laminated. Note that a polyacrylonitrile layer, an aluminum layer and a polyethylene terephthalate layer refer to layers containing polyacrylonitrile, aluminum and polyethylene terephthalate, respectively as a main component. The phrase "containing ... as a main component" used in the present invention means that polyacrylonitrile, aluminum or polyethylene terephthalate is contained in each layer in an amount of 50 mass% or more. Polyacrylonitrile in a polyacrylonitrile layer is preferably 90 mass% or more, and more preferably, a polyacrylonitrile layer consists of polyacrylonitrile. Aluminum in an aluminum layer is preferably 90 mass% or more, and more preferably, an aluminum layer consists of aluminum. Polyethylene terephthalate in a polyethylene terephthalate layer is preferably 90 mass% or more, and more preferably, a polyethylene terephthalate layer consists of polyethylene terephthalate. Other components may be appropriately blended into each layer.

Traditionally, used are a packaging container composed of a multilayered film comprising PAN and AL (For example, see Japanese Patent No. 5093545) and a packaging container composed of a multilayered film comprising a polyethylene layer (hereinafter, the polyethylene layer is also referred to as "PE") and the like, but not comprising PAN as a storage container of a rivastigmine-containing adhesive patch. However, the present inventors find that an alteration of rivastigmine in an adhesive patch can be better controlled as compared with the conventionally used packaging containers when a packaging container composed of a multilayered film in which at least PAN, AL and PET are laminated.

There is no particular limitation for the packaging container according to the present invention as long as it comprises a multilayered film in which at least PAN, AL and PET are laminated.

PAN is preferably used for the innermost layer of a packaging container (i.e., a layer in a multilayered film of a packaging container which directly makes contact with an adhesive patch enclosed inside the packaging container) in view of that it can prevent rivastigmine from being adsorbed by the packaging container while further increasing an alteration controlling effect of rivastigmine. Further, PAN well functions as a sealant layer of a packaging container. AL, which has a function to block the penetration of light and oxygen into a package body, preferably does not make direct contact with an adhesive patch, i.e., preferably is not the innermost layer of a packaging container in view of preventing rivastigmine from being adsorbed by the packaging container. Further, AL is preferably not provided at the outermost layer of a packaging container since it is relatively easily damaged. Further, PET is preferably provided at the outermost layer of a packaging container since it can well function as a surface base material while further increasing an alteration controlling effect of rivastigmine. Therefore, the packaging container according to the present invention preferably comprises a multilayered film in which PAN, AL and PET are laminated in this order from the innermost layer. However, in a multilayered film in which PAN, AL and PET are laminated in this order, other layers may be provided between and/or outside each layer of PAN, AL and PET as long as the object of the present invention is not impaired. For example, the packaging container may comprise a multilayered film in which a known sealant layer/PAN/an anti-oxidant layer/AL/PET/ a known surface base material are laminated in this order from the innermost layer.

Multiple PANs, ALs and PETs may be contained in the multilayered film for each. For example, the multilayered film may have a configuration such as PAN/AL/PAN(PET)/PET (PAN), PAN/PET/AL/PAN (PET) and the like in these orders from the innermost layer. Other layers may be provided between and/or outside each layer as long as the object of the present invention is not impaired.

There is no particular limitation for the thicknesses of PAN, AL and PET of a multilayered film, but the thicknesses of PAN, AL and PET may be 10 to 50 μm (preferably 20 to 40 μm), 1 to 15 μm (preferably 5 to 9 μm) and 5 to 20 μm (preferably 10 to 13 μm), respectively. There is no particular limitation for the layer ratio of PAN, AL and PET, but PAN is preferably prepared to be thicker in view of an enhanced alteration controlling effect of rivastigmine. An example of the layer ratio of PAN, AL and PET is the thickness of PAN : the thickness of AL : the thickness of PET = 30:7:12. The layer ratio is 1:0.1-0.3:0.3-0.5 when the thickness of PAN is taken as 1. In a case where multiple PANs, ALs and PETs are contained in a multilayered film for each, the thickness of each layer of PANs, ALs and PETs can be adjusted so that the total value of the thickness of each layer falls within the aforementioned corresponding range. Note that the thickness of each layer of the multilayered film may be determined by the conventionally known method, and the average value of the values obtained may be adjusted within the aforementioned range.

Layers other than PAN, AL, and PET (for example, a polyethylene layer, a paper layer, a known oxygen-absorbing layer, a known moisture-absorbing layer and the like) may be contained in the multilayered film. The thicknesses of these layers may be appropriately adjusted depending on the desired effect.

There is no particular limitation for the method of manufacturing a multilayered film, and known laminating methods can be used such as the dry lamination method, the extrusion lamination method and the wet lamination method.

There is no particular limitation for the method of manufacturing a packaging container from a multilayered film, and known methods can be used such as a seal by heat sealing.

(Configuration of adhesive patch)

The adhesive patch according to the present invention comprises a support and an adhesive layer containing an adhesive and rivastigmine and disposed on the above support. The adhesive patch may be those in which a known skin adhesive layer (polyisobutylene and the like), controlled-release membrane and strippable liner (an oxygen impermeable liner and the like) are laminated to an adhesive layer.

[Rivastigmine]

As rivastigmine in the present invention, free rivastigmine which shows an acetylcholineaterase inhibitory action and/or pharmaceutically acceptable salts thereof can be used. Salts of rivastigmine include rivastigmine hydrochloride, rivastigmine tartrate and the like. Among these, free rivastigmine is preferred in view of good skin permeability.

There is no particular limitation for the content of rivastigmine in an adhesive layer, and it can be appropriately adjusted depending on the desired therapeutic effect. For example, rivastigmine may be contained in an adhesive layer in an amount of 0.1 mass% or more, preferably 1 mass% or more, more preferably 10 mass% or more. Further, rivastigmine may be contained in an adhesive layer in an amount of 40 mass% or less, preferably less than 30 mass%, more preferably 25 mass% or less, most preferably 20 mass% or less.

[Adhesive]

There is no particular limitation for the adhesive contained in an adhesive layer as long as it is commonly used for an adhesive patch applied to the skin, and it may be one or more of acrylic adhesives, rubber based adhesives or silicon based adhesives.

Among the above adhesives, acrylic adhesives are preferred in view of that transdermal absorption of rivastigmine is less reduced. Among acrylic adhesives, an acrylic adhesive substantially free from a carboxyl group (a carboxy group) in view of that it is less reactive with rivastigmine, and can control the decrease in transdermal absorption of rivastigmine, and is less affected by oxygen. The phrase "substantially free from a carboxyl group" refers to not only those not containing a carboxyl group at all but also those in which carboxyl groups are all theoretically replaced with substituents such as ester linkages. These include, for example, a case in which a trace amount of ester linkages and the like are converted into free carboxyl groups due to hydrolysis, and a case in which free carboxyl group is included as impurities from a raw material.

There is no particular limitation for the acrylic adhesive substantially free from a carboxyl group, and, for example, adhesives described in Japanese Unexamined Patent Application No. 2005-325101, Japanese Patent No. 3809462 and the like can be used. Among these, in particular, the acrylic adhesive described in Japanese Unexamined Patent Application No. 2005-325101 can be suitably used.

As the acrylic adhesive in the present invention, an acrylic adhesive substantially free from a carboxyl group is preferred in view of that it is less reactive with rivastigmine, and can better control the decrease in transdermal absorption of rivastigmine, and is less affected by oxygen. Further, an acrylic adhesive substantially free from a carboxyl group and a hydroxy group is particularly preferred.

Other components other than those described above may be contained in an adhesive patch, if desired. Such components include adhesiveness-conferring agents, plasticizing agents, antiseptic agents, pH adjusters, chelating agents, transdermal absorption promoting agents, excipients, flavoring agents, coloring agents, fatty acids, fats and oils and the like.

Inside the package body according to the present invention, the stability of rivastigmine in an adhesive patch is excellent even in a case where an anti-oxidant (agents commonly used for preventing oxidation of a drug in an adhesive patch such as dibutylhydroxytoluene, vitamin E, butylated hydroxyanisole and propyl gallate) is not blended. However, an anti-oxidant may be blended into an adhesive patch, if desired.

(Method of manufacturing adhesive patch)

There is no particular limitation for the method of preparing an adhesive patch, and known preparing methods can be used for preparing an adhesive patch. They include, for example, a method of obtaining an adhesive patch, comprising uniformly stirring components of an adhesive layer, coat-drying the resulting composition onto a surface of a support using a coater.

(Method of manufacturing package body)

The package body according to the present invention can be obtained by enclosing an adhesive patch into a packaging container, and sealing the packaging container. When enclosing, an adhesive patch may be placed into a packaging container which is then sealed after adjusting the amount of oxygen gas and the like inside the packaging container. Alternatively, an adhesive patch may be placed into a packaging container which is then sealed as it is. For sealing, known methods can be used such as heat sealing and sealing with adhesives.

In the case of the package body according to the present invention, an alteration of rivastigmine in an adhesive patch can be controlled without enclosing an anti-oxidant and an oxygen scavenger (agents commonly used for reducing the amount of oxygen inside a package body, such as iron powder, zinc powder, hydrosulfite, ascorbic acid-based agents, polyhydric alcohol-based agents and activated carbon-based agents) into the package body as a separate member distinct from the adhesive patch or a member integrated with the adhesive patch, or without performing inert gas substitution (nitrogen substitution and the like) before enclosing the package body. The package body according to the present invention may consist of a packaging container and an adhesive patch enclosed in the above packaging container. However, enclosing an anti-oxidant and/or an oxygen scavenger and performing inert gas substitution shall not be excluded from the scope of the present invention.

Stability of adhesive patch in package body

Unlike the conventional package bodies, in the package body according to the present invention, the stability of a drug in an adhesive patch is good without enclosing an anti-oxidant and an oxygen scavenger along with the adhesive patch or without performing inert gas substitution and the like. The stability of a drug in an adhesive patch can be determined by detecting an analog of the drug with HPLC. An analog of a drug is defined as a substance produced when the drug is altered and the like due to oxygen, which shows reduced or no efficacy of the original drug. Therefore, a lower amount of an analog detected indicates that the stability of the drug is high.

The stability of a drug in an adhesive patch can also be determined by the temporal change of color tone of the adhesive patch. The color tone of an adhesive patch may be determined with a color difference meter.

Stability of rivastigmine in adhesive patch

In the case of the package body according to the present invention, an alteration of rivastigmine in an adhesive patch can be controlled, and the drug stability is good without enclosing an anti-oxidant and an oxygen scavenger along with the adhesive patch or without performing inert gas substitution and the like. The term "alteration of rivastigmine" used in the present invention means that the amount of rivastigmine is decreased, or a degradation products, an analog of rivastigmine and the like are increased due to decomposition and the like of rivastigmine. Whether an alteration of rivastigmine in an adhesive patch is controlled or not can be determined by determining rivastigmine or an analog thereof and the like in the adhesive patch by HPLC and the like. An analog of rivastigmine is defined as a substance produced when rivastigmine is altered and the like due to oxygen or light which shows reduced or no drug efficacy of the original rivastigmine. Therefore, a lower amount of an analog detected in an adhesive patch indicates that the drug stability of rivastigmine is high.

EXAMPLES

Below, the present invention will be described in more detail with reference to Examples, but the present invention shall not be limited to these.

Example 1: Evaluation of stability of nicotine in nicotine-containing adhesive patch inside package body

An acrylic adhesive, nicotine and the like were blended according to a rate shown in "Table 1" to prepare a composition, which was then coat-dried (60°C, 15 minutes) onto a support (a PET film is used) to prepare a drug-containing adhesive layer. A controlled-release membrane and a skin adhesive layer comprising polyisobutylene were laminated on the drug-containing adhesive layer, and a strippable liner was further laminated on the skin adhesive layer to obtain an adhesive patch. Further, an anti-oxidant was blended into some of the formulations in a rate shown in "Table 1."

Note that the acrylic adhesive used in this Example is described in Japanese Unexamined Patent Application No. 2005-325101 (specifically, an adhesive obtained by mixing a solution obtained in Example 3 of preparing the copolymer (A) and a solution obtained in Example 3 of preparing the copolymer (B) in a mass ratio of 100:5 (the copolymer (A): the copolymer (B)).

Each adhesive patch obtained was individually placed into a bag in which polyacrylonitrile/aluminum/polyethylene terephthalate are laminated (which corresponds to the packaging container according to the present invention, and hereinafter, referred to as a "PAN/Al/PET bag"). One of the following treatments was performed when the adhesive patch was placed into a PAN/Al/PET bag. In the followings, the package body obtained via the treatment (2) corresponds to the package body according to the present invention.

(1) The adhesive patch is placed into a bag, and the bag is sealed as it is ("Normal" in Table 1).
(2) The adhesive patch is placed into a bag, and the bag is then sealed after the concentration of oxygen gas in the bag is adjusted to 20.6 vol%, and the amount of oxygen gas is adjusted to 7 μL using a vacuum pump ("Vacuum" in Table 1).

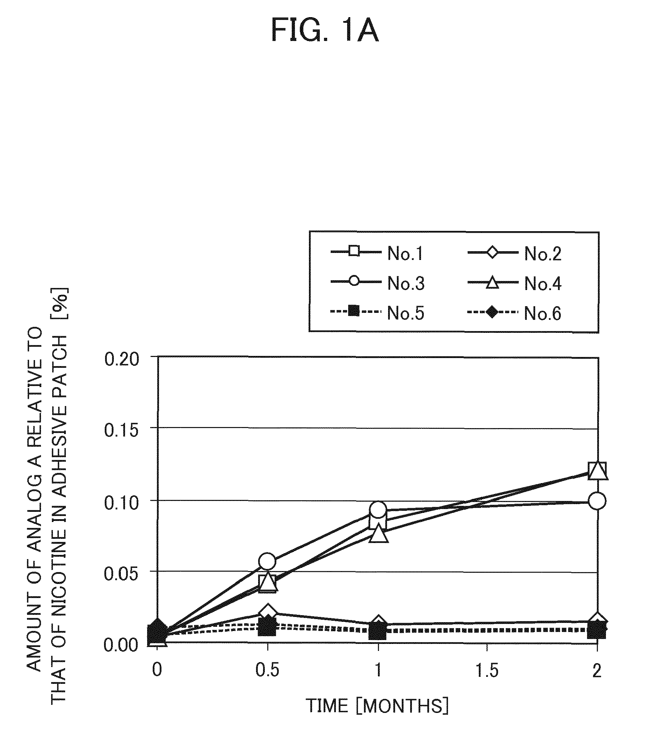

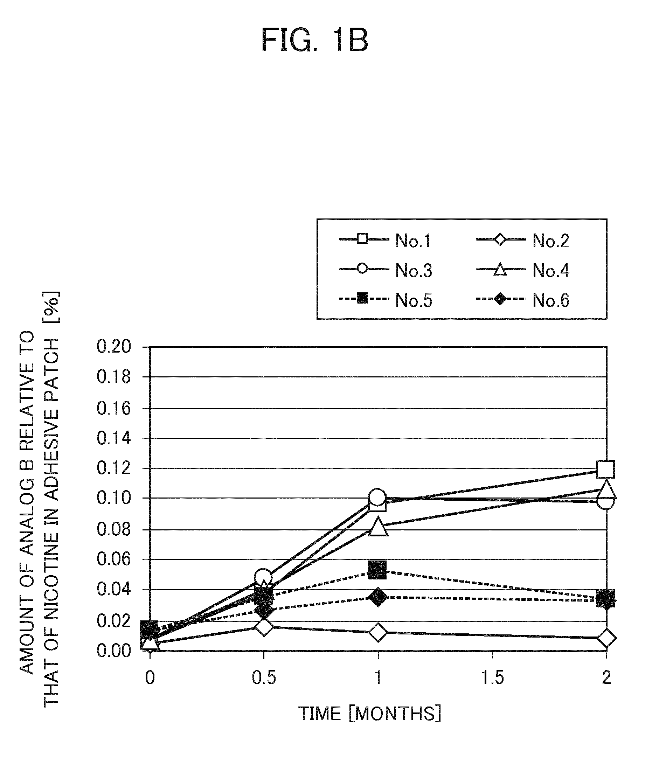

The sealed adhesive patch was stored at 60°C (a humidity of 75%). The content of a nicotine analog in the adhesive patch was measured by HPLC (UV 254 nm) at each time of: upon the start of storage, 0.5 months after the start of storage, 1 month after the start of storage, 1.5 months after the start of storage, 2 months after the start of storage. The results are shown in Fig. 1. Note that Analog A in Fig. 1A corresponds to a peak detected at 1.95 minutes in HPLC. Further, Analog B in Fig. 1B corresponds to a peak detected at 20.16 minutes in HPLC. Note that nicotine is detected at 15.4 minutes in HPLC.

[Table 1]

| No. | Composition of drug-containing adhesive layer (Mass%) | | | | Treatment |
| --- | --- | --- | --- | --- | --- |
| | Nicotine | Acrylic adhesive | Lactic acid | Anti-oxidant (Dibutylhydroxytoluene) | |
| 1 | 42 Mass% | 56.8 Mass% | 0.2 Mass% | 1 Mass% | Normal |
| 2 | | | | | Vacuum |
| 3 | | | | | Normal |
| 4 | | | | | |
| 5 | | 57.8 Mass% | | None | |
| 6 | | 58 Mass% | None | None | Vacuum |

As shown in Fig. 1, the generation of analogs of nicotine was controlled in the adhesive patch inside the package bodies where the amount and concentration of oxygen gas were adjusted to the ranges according to the present invention (No. 2, 5 and 6). That is, it is revealed that an alteration of nicotine is controlled in the package body according to the present invention, leading to a good drug stability.

Example 2: Evaluation of color tone of nicotine-containing adhesive patch inside package body

An adhesive patch was obtained as in Example 1 except that a composition was prepared by blending components according to a rate shown in "Table 2."

Each adhesive patch obtained was individually placed into a PAN/Al/PET bag. One of the following treatments was performed when the adhesive patch was placed into a PAN/Al/PET bag.

(1) The adhesive patch is placed into a bag and the bag is sealed as it is ("Normal" in Table 2).
(2) The adhesive patch is placed into a bag, and the bag is sealed after the concentration of oxygen gas in the bag is adjusted to 20.6 vol%, and the amount of oxygen gas is adjusted to 7 μL using a vacuum pump ("Vacuum" in Table 2).

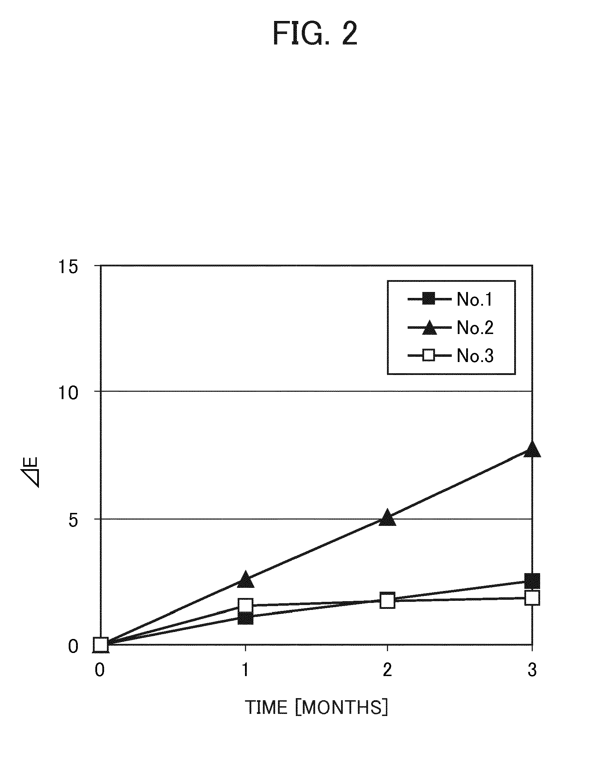

The sealed adhesive patch was stored at 60°C (a humidity of 75%). The color tone of the adhesive patch was measured in accordance with the following method at each time of: upon the start of storage, 1 month after the start of storage, 2 months after the start of storage, 3 months after the start of storage. The results are shown in Fig. 2.

(Study of tone change of adhesive patch)

The chromaticity of the adhesive patch (this can be considered to be the same as the chromaticity of a drug-containing adhesive layer) was measured using a color difference meter, and the tone change of the adhesive patch was computed as follows.
The color tone of the adhesive patch was determined based on the "a-scale" for the degree of red-green, the "b-scale" for the degree of blue-yellow and the "L-scale" for the degree of white-black. Subsequently, the degrees of the tone change before and after the start of storage were expressed in the square root of sum of squares ΔE of the difference in each scale before and after the start of storage (represented by the following formula).

$$\Delta E \left(= \left( \left( \Delta a \right)^2 + \left( \Delta b \right)^2 + \left( \Delta L \right)^2 \right)^{1/2} \right)$$

[Table 2]

| No. | Composition of drug-containing adhesive layer (Mass%) | | | | Treatment |
|---|---|---|---|---|---|
| | Nicotine | Acrylic adhesive | Lactic acid | Anti-oxidant (Dibutylhydroxytoluene) | |
| 1 | 42 Mass% | 57.3 Mass% | 0.2 Mass% | 0.5 Mass% | Normal |
| 2 | | 57.6 Mass% | | 0.2 Mass% | Normal |
| 3 | | 57.8 Mass% | | None | Vacuum |

As shown in Fig. 2, the change over time in the color tone was controlled in the package body where the amount and concentration of oxygen gas were adjusted to the ranges according to the present invention (No. 3). That is, it is revealed that the stability of an adhesive patch is high without using an anti-oxidant in the package body according to the present invention.

Example 3: evaluation of stability of rivastigmine in rivastigmine-containing adhesive patch inside package body

A composition was prepared by blending 84 parts by mass of an acrylic adhesive and 16 parts by mass of rivastigmine in this rate, and this was coat-dried (60°C, 15 minutes) onto a support (a PET film was used) to obtain an adhesive patch.
Note that the acrylic adhesive used in this Example is described in Japanese Unexamined Patent Application No. 2005-325101 (specifically, an adhesive obtained by mixing a solution obtained in Example 3 of preparing the copolymer (A) and a solution obtained in Example 3 of preparing the copolymer (B) in a mass ratio of 100:5 (the copolymer (A): the copolymer (B)).
Each adhesive patch obtained was individually placed into a PAN/Al/PET bag. One of the following treatments was performed when the adhesive patch was placed into a PAN/Al/PET bag.

(1) The adhesive patch is placed into a bag, and the bag is sealed as it is ("Normal" in Table 3).
(2) The adhesive patch is placed into a bag, and the bag is sealed after the concentration of oxygen gas in the bag is adjusted to 18.8 vol%, and the amount of oxygen gas is adjusted to 4 μL using a vacuum pump ("Vacuum" in Table 3).

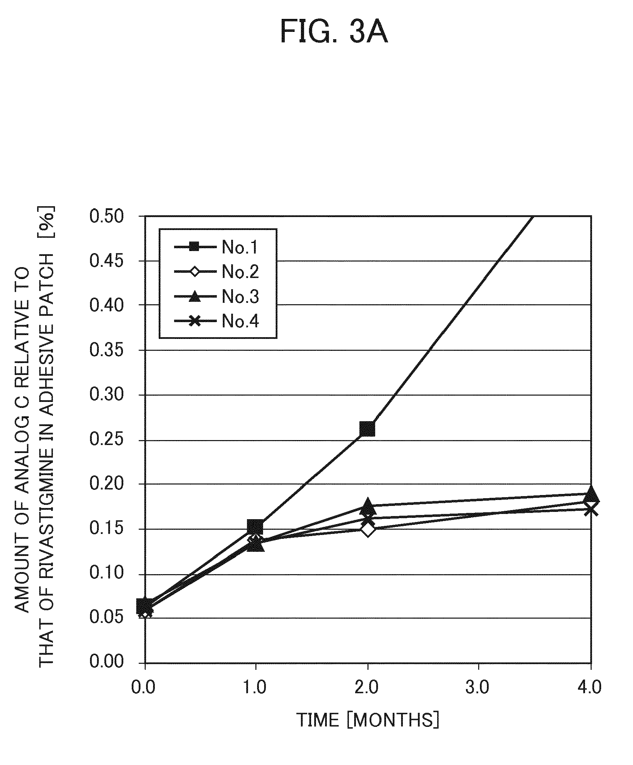

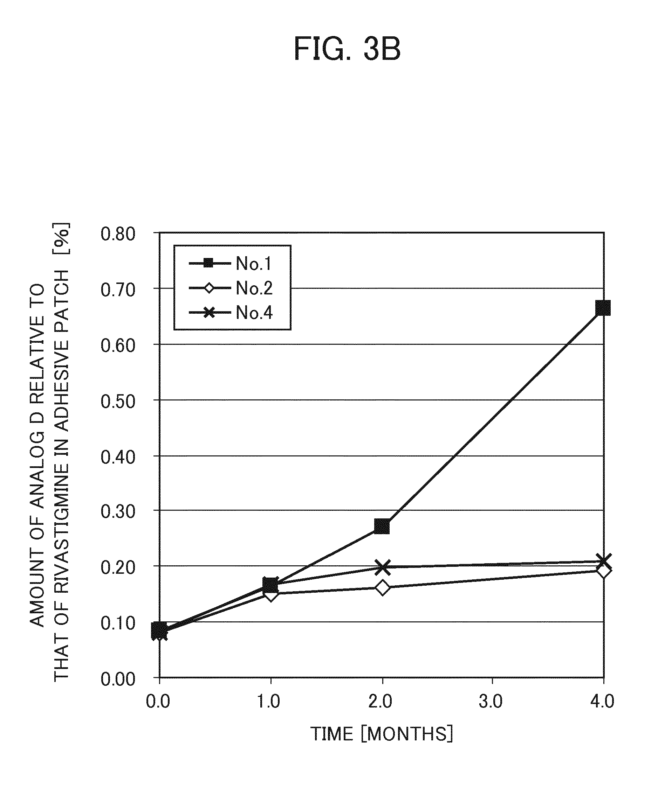

The sealed adhesive patch was stored at 60°C (a humidity of 75%). The content of a rivastigmine analog in the adhesive patch was measured by HPLC (UV 215 nm) at each time of: upon the start of storage, 1 month after the start of storage, 2 months after the start of storage, 3 months after the start of storage, 4 months after the start of storage. The results are shown in Fig. 3. Note that Analog C in Fig. 3A corresponds to a peak detected at 20.4 minutes in HPLC. Further, Analog D in Fig. 3B corresponds to a peak detected at 72.3 minutes in HPLC. Note that rivastigmine is detected at 10.2 minutes in HPLC.

[Table 3]

| No. | Anti-oxidant | Treatment |
|---|---|---|
| 1 | None | Normal |
| 2 | None | Vacuum |
| 3 | None | Vacuum |
| 4 | None | Vacuum |

As shown in Fig. 3, the generation of rivastigmine analogs in the adhesive patch was controlled in the package body where the amount and concentration of oxygen gas were adjusted to the ranges according to the present invention (No. 2 to 4). That is, it is revealed that an alteration of rivastigmine is controlled in the package body according to the present invention, leading to a good stability. Example 4: Study about the amount and concentration of oxygen gas inside package body - 1

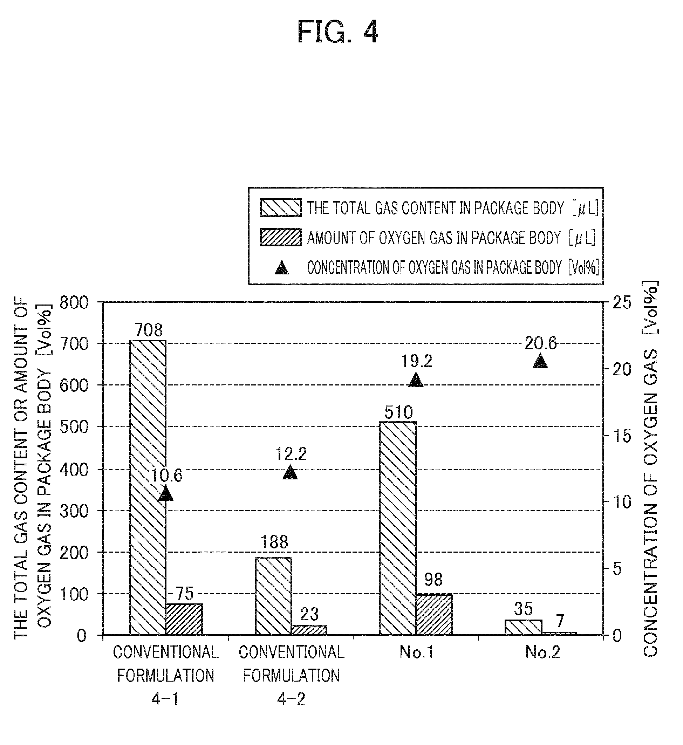

The total gas content, the amount of oxygen gas and the concentration of oxygen gas inside a package body were measured for the package bodies No. 1 and No. 2 prepared in Example 1 and the conventional package bodies (in which a nicotine-containing adhesive patch is enclosed). The results are shown in Fig. 4.

Note that the total gas content inside the package body was determined as follows: an adhesive sheet was attached to the package body in order to prevent gas leakage; piercing each package body (at a place where the adhesive sheet was attached) with a micro syringe under water; then withdrawing air inside the package body by pulling the plunger; and reading a scale on the syringe to determine the total gas content inside the package body when the plunger stopped. The concentration of oxygen gas in the package body was measured with a needle type micro oxygen gas concentration meter (Product name "Microx TX3", Taitec Corporation) pierced into each package body. The amount of oxygen gas inside the package body was computed from the concentration of oxygen gas and the total gas content.

As shown in Fig. 4, the total gas content inside the package body decreases when a vacuum pump is used. However, the ratio of oxygen inside the package body is about 20 vol%, which is substantially the same as in a case where a vacuum pump is not used (that is, the ratio of oxygen in the normal atmospheric composition) (No. 1 and 2).

Example 5: Study about the amount and concentration of oxygen gas inside package body - 2

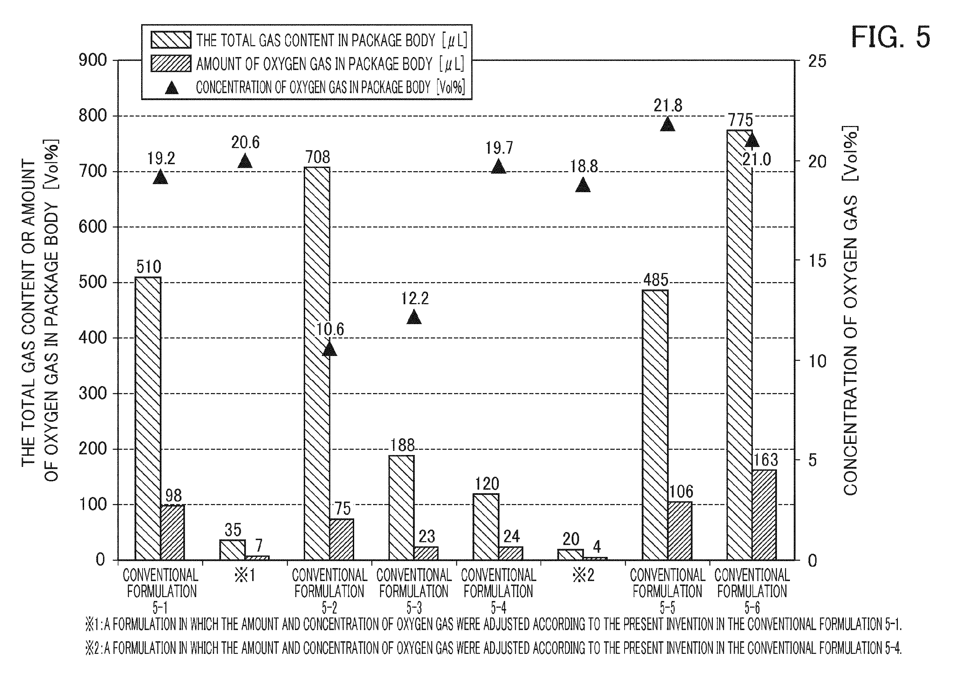

The total gas content, the amount of oxygen gas and the concentration of oxygen gas inside the package body were measured as in Example 4 for the package bodies according to the present invention and the conventional package bodies (those in which a nicotine-containing adhesive patch or a rivastigmine-containing adhesive patch was enclosed). The results are shown in Fig. 5. Note that the "conventional formulation 5-1" and "*1" in Fig. 5 are similar to the package bodies of No. 1 and No. 2 prepared in Example 1, respectively. Further, the "conventional formulation 5-4" and "*2" are similar to the package bodies of No. 1 and No. 2 prepared in Example 3, respectively. Moreover, an anti-oxidant was blended into the adhesive patches of the conventional formulations 5-2, 5-3, 5-5 and 5-6.

As shown in Fig. 5, the total gas content inside the package body decreases when a vacuum pump is used. However, the ratio of oxygen inside the package body is about 20 vol%, which is substantially the same as in a case where a vacuum pump is not used (that is, the ratio of oxygen in the normal atmospheric composition). Further, it appeared that the present invention has a lower amount of oxygen gas inside a package body than the conventional formulation, and an alteration of a drug in an adhesive patch due to oxygen can be more efficiently controlled without using an oxygen scavenger, an anti-oxidant or the like.

Example 6: Evaluation of stability of rivastigmine in rivastigmine-containing adhesive patch inside package body - I

An acrylic adhesive and rivastigmine were blended according to a rate shown in Table 4 to prepare a composition, which was then coat-dried (60°C, 15 minutes) onto a support to prepare a rivastigmine-containing adhesive patch. Note that a PET film was used as the support.

Note that the acrylic adhesive used in this Example was prepared as follows. That is, a solution of the copolymer (A) and a solution of the copolymer (B) obtained by the synthesis method shown below were mixed at a mass ratio of 100:5 (the copolymer (A) : the copolymer (B)) to obtain an acrylic adhesive.

[Copolymer (A)]

Added and mixed were 200 parts by mass of acrylic acid 2-ethylhexyl, 100 parts by mass of butyl acrylate, 50 parts by mass of diacetone acrylamide and 300 parts by mass of ethyl acetate. This mixture was transferred into a separable flask equipped with a stirrer and a reflux condenser, and heated to 75°C with stirring and performing nitrogen substitution. A solution in which 2 parts by mass of benzoyl peroxide was dissolved in 20 parts by mass of ethyl acetate was aliquoted into 5 parts, and one of them was added to the separable flask to start polymerization. The remaining 4 aliquots were added by 1 aliquot at 1 hour intervals staring from 2 hours after the reaction started. The reaction was then allowed for additional 2 hours after the completion of the addition. Note that in order to adjust viscosity, ethyl acetate was added every 2 hours for 4 times each time in an amount of 50 parts by mass after the start of the reaction. This was cooled after the reaction completed, and ethyl acetate was then added to obtain the copolymer (A) with a solid content concentration of 30 mass%.

[Copolymer (B)]

Added and mixed are 660 parts by mass of ethyl acrylate, 70 parts by mass of diacetone acrylamide, 40 parts by mass of dodecylmercaptan as a molecular weight modifier and 400 parts by mass of ethyl acetate. This mixture was provided in a separable flask equipped with a stirrer and a reflux condenser, and heated to 70°C with stirring and performing nitrogen substitution. A solution in which 5 parts by mass of azobisisobutyronitrile was dissolved in 100 parts by mass of ethyl acetate was aliquoted into 5 parts, and one of them was added to the separable flask to start polymerization. The remaining 4 aliquots were added by 1 aliquot at 1 hour intervals staring from 2 hours after the reaction started. The reaction was then allowed for additional 2 hours after the completion of the addition. Note that ethyl acetate was added every 2 hours for 3 times each time in an amount of 50 parts by mass after the reaction started in order to adjust viscosity. Then, added was a solution in which 40 parts by mass of adipic acid dihydrazide was dissolved in a mixed liquid of 40 parts by mass of purified water, 1600 parts by mass of methanol and 260 parts by mass of ethyl acetate, and 5 parts by mass of concentrated hydrochloric acid was further added, and then heated to 70°C. This was cooled after the reaction completed, and washed 3 times with purified water. Then, the product was dissolved in a mixed solvent of 700 parts by mass of ethyl acetate, 1400 parts by mass of acetone and 400 parts by mass of methanol to obtain the copolymer (B) with a solid content concentration of 30 mass%.

[Table 4]

| Composition of rivastigmine-containing adhesive patch (Mass%) | | |
|---|---|---|
| Adhesive patch number | Acrylic adhesive | Rivastigmine |
| 1~5 | 84 | 16 |
| 6 | Exelon® patch (commercially available product) | |

Each adhesive patch obtained was individually placed into a packaging container comprising a corresponding multilayered film having a configuration shown in Table 5, which was then directly sealed to obtain a package body. Note that the term "multilayered film configuration" shown in Table 5 refers to a lamination order of layers, and the layer shown in the left most side corresponds to the innermost layer of a packaging container (that is, a layer which makes contact with an adhesive patch enclosed inside a packaging container). For example, for the package body No. "1" in Table 5, a multilayered film was used in which the innermost layer was PAN, and AL and PET were then laminated in this order, and the outermost layer was PET.

[Table 5]

| Package body number | Adhesive patch number | Multilayered film configuration |
|---|---|---|
| 1 | 1 | PAN(30$\mu$m)/AL(7$\mu$m)/PET(12$\mu$m) |
| 2 | 2 | PE(30$\mu$m)/AL(7$\mu$m)/PE(15$\mu$m)/PET(12$\mu$m) |
| 3 | 3 | PE/oxygen absorbing layer/AL/PET, Product name Oxyguard (Toyo Seikan Co., Ltd.) |

EP 2 939 667 A1

(continued)

| Package body number | Adhesive patch number | Multilayered film configuration |
|---|---|---|
| 4 | 4 | PE/moisture absorbing layer/AL/PET, Product name Moisture Guard (Toyo Seikan Co., Ltd.) |
| 5 | 5 | Moisture-absorbing sealant layer (25 μm)/AL (7 μm)/PE(15 μm)/PET (12 μm), moisture-absorbing packaging pouch from Dai Nippon Printing Co., Ltd. |
| 6 | 6 | Exelon® patch (commercially available product) |

Each package body was stored at 60°C (a humidity of 75%). The content of rivastigmine in the adhesive patch was measured by HPLC at each time of: upon the start of storage (Initial), 2 weeks after the start of storage (2w), 1 month after the start of storage (1M), 2 months after the start of storage (2M). The results are shown in Table 6. Note that the measurement results in Table 6 show a relative value when Initial is taken as "100."

[Table 6]

| | | 60°C | | |
|---|---|---|---|---|
| Package body number | Initial | 2w | 1M | 2M |
| 1 | 100.00 | 96.14 | 97.41 | 100.27 |
| 2 | 100.00 | 94.92 | 93.86 | 93.39 |
| 3 | 100.00 | 93.95 | 92.66 | 86.71 |
| 4 | 100.00 | 95.20 | 91.54 | 91.42 |
| 5 | 100.00 | 97.11 | 95.56 | 95.52 |
| 6 | 100.00 | 98.60 | 97.10 | 94.30 |

Stability evaluation of rivastigmine (table title)

As shown in Table 6, the package body according to the present invention (package body No. 1) showed less variation from the start of storage in the content of rivastigmine in the adhesive patch even after long storage, showing that an alteration of rivastigmine was controlled. In contrast, for the package bodies No. 2 to No. 5 which do not contain PAN, AL and PET in their multilayered film configurations, the content of rivastigmine in the adhesive patch was decreased, and an alteration of rivastigmine was observed after long storage. Example 7: Evaluation of stability of rivastigmine in rivastigmine-containing adhesive patch inside package body - II

Two rivastigmine-containing adhesive patches were prepared as in Example 6. Each adhesive patch obtained was individually placed into a packaging container comprising a corresponding multilayered film having a configuration shown in Table 7, which was then directly sealed to obtain a package body. Note that the term "multilayered film configuration" shown in Table 7 refers to, similar to Table 5, the lamination order of layers, and the layer shown in the left most side corresponds to the innermost layer of a packaging container.

[Table 7]

| Package body number | Adhesive patch number | Multilayered film configuration |
|---|---|---|
| 1 | 1 | PAN(30μm)/AL(7μm)/PET(12 μm) |
| 2 | 2 | PE(30μm)/AL(7μm)/PE(15μm)/PET(12μm) |

Each package body was stored at 60°C or 40°C (a humidity of 75%). The content of rivastigmine analogs in the adhesive patch was measured by HPLC (UV 254 nm) at each time of: upon the start of storage (Initial), 2 weeks after the start of storage, 1 month after the start of storage, 2 months after the start of storage or 3 months after the start of storage. The results are shown in a Tables 8 to 10. Note that a lower amount of the detected rivastigmine analogs indicates that drug stability of rivastigmine in the adhesive patch is high. Note that rivastigmine is detected at a retaliation time of 10.2 minutes by HPLC (UV 254 nm).

13

[Table 8]

| Analog 1 (Retention time RT: 5.5 minutes) | | | | | | |
|---|---|---|---|---|---|---|
| Adhesive patch number | Initial | 60°C | | | 40°C | |
| | | 2 w | 1 M | 2M | 1 M | 3M |
| 1 | 0.02 | 0.05 | 0.06 | 0.09 | 0.03 | 0.07 |
| 2 | 0.02 | 0.05 | 0.08 | 0.13 | 0.03 | 0.07 |

[Table 9]

| Analog 2 (Retention time RT: 20.4 minutes) | | | | | | |
|---|---|---|---|---|---|---|
| Adhesive patch number | Initial | 60°C | | | 40°C | |
| | | 2 w | 1 M | 2 M | 1 M | 3 M |
| 1 | 0.07 | 0.14 | 0.24 | 0.58 | 0.11 | 0.16 |
| 2 | 0.07 | 0.21 | 0.41 | 1.13 | 0.11 | 0.20 |

[Table 10]

| Analog 3 (Retention time RT: 72.3 minutes) | | | | | | |
|---|---|---|---|---|---|---|
| Adhesive patch number | Initial | 60°C | | | 40°C | |
| | | 2 w | 1 M | 2 M | 1 M | 3 M |
| 1 | 0.07 | 0.14 | 0.27 | 0.70 | 0.13 | 0.21 |
| 2 | 0.07 | 1.20 | 2.01 | 3.76 | 0.74 | 1.58 |

As shown in Tables 8 to 10, for the package body according to the present invention (package body No. 1), the generation of rivastigmine analogs in the adhesive patch was controlled, and an alteration of rivastigmine was controlled after long storage. In contrast, for the package body No. 2 which does not contain PAN, AL and PET in the multilayered film configuration, the amount of generated analogs of rivastigmine in the adhesive patch was increased, and an alteration of rivastigmine was observed after long storage. Such alteration was significantly observed in particular when stored at high temperature (60°C).

**Claims**

1. A package body comprising a packaging container and a drug-containing adhesive patch enclosed inside the packaging container,
   wherein the amount of oxygen gas inside the package body is 20 $\mu$L or less, and the concentration of oxygen gas is 17.0 to 25.0 vol%.

2. The package body according to claim 1, wherein the package body does not contain an oxygen scavenger as a separate member distinct from the drug-containing adhesive patch.

3. The package body according to claim 1 or 2, wherein the amount of oxygen gas is 4 $\mu$L/mg or less per amount of a drug in the drug-containing adhesive patch.

4. The package body according to any one of claims 1 to 3, wherein the drug is any of nicotine, rivastigmine or pharmacologically acceptable salts thereof.

5. The package body according to any one of claims 1 to 4, wherein the drug-containing adhesive patch comprises a support and an adhesive layer disposed on the support, the adhesive layer comprising an acrylic adhesive substantially free from a carboxyl group.

6. A package body comprising a packaging container and an adhesive patch enclosed inside the packaging container, wherein the adhesive patch comprises a support and an adhesive layer containing a drug and disposed on the support, the drug comprises at least rivastigmine and/or a pharmaceutically acceptable salt thereof, the packaging container comprises a multilayered film in which at least a polyacrylonitrile layer, an aluminum layer and a polyethylene terephthalate layer are laminated.

7. The package body according to claim 6, wherein the innermost layer of the packaging container is a polyacrylonitrile layer.

8. The package body according to claim 7, wherein the multilayered film is a multilayered film in which a polyacrylonitrile layer, an aluminum layer and a polyethylene terephthalate layer are laminated in this order.

9. The package body according to any one of claims 6 to 8, wherein the polyacrylonitrile layer has a thickness of 10 to 50 $\mu$m, and the aluminum layer has a thickness of 1 to 15 $\mu$m, and the polyethylene terephthalate layer has a thickness of 5 to 20 $\mu$m.

10. The package body according to any one of claims 6 to 9, wherein the package body does not contain any one or more of an oxygen scavenger and an anti-oxidant.

11. The package body according to any one of claims 6 to 10, wherein the adhesive layer comprises an acrylic adhesive.

# FIG. 1A

# FIG. 1B

# FIG. 2

# FIG. 3A

# FIG. 3B

# FIG. 4

FIG. 5

EP 2 939 667 A1

※1: A FORMULATION IN WHICH THE AMOUNT AND CONCENTRATION OF OXYGEN GAS WERE ADJUSTED ACCORDING TO THE PRESENT INVENTION IN THE CONVENTIONAL FORMULATION 5-1.

※2: A FORMULATION IN WHICH THE AMOUNT AND CONCENTRATION OF OXYGEN GAS WERE ADJUSTED ACCORDING TO THE PRESENT INVENTION IN THE CONVENTIONAL FORMULATION 5-4.

# EP 2 939 667 A1

<table>
<tr><td colspan="2" align="center">INTERNATIONAL SEARCH REPORT</td><td>International application No.<br>PCT/JP2013/084950</td></tr>
</table>

**A. CLASSIFICATION OF SUBJECT MATTER**
*A61K9/70(2006.01)i, A61J1/00(2006.01)i, A61K31/27(2006.01)i, A61K31/465 (2006.01)i, A61K47/32(2006.01)i, A61M37/00(2006.01)i, A61P25/28(2006.01)i, A61P25/34(2006.01)i*
According to International Patent Classification (IPC) or to both national classification and IPC

**B. FIELDS SEARCHED**

Minimum documentation searched (classification system followed by classification symbols)
A61K9/70, A61J1/00, A61K31/27, A61K31/465, A61K47/32, A61M37/00, A61P25/28, A61P25/34

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2012-51875 A  (Hisamitsu Pharmaceutical Co., Inc.),<br>15 March 2012 (15.03.2012),<br>claims 1 to 2; paragraphs [0001], [0006] to [0009], [0031] to [0046]<br>& US 2012/0031047 A1 | 1-11 |
| Y | JP 2009-39210 A  (Hosokawa Yoko Co., Ltd.),<br>26 February 2009 (26.02.2009),<br>claim 1; paragraphs [0001], [0004] to [0007], [0013] to [0041]<br>(Family: none) | 1-5 |
| Y | JP 2006-346888 A  (Kyodo Printing Co., Ltd.),<br>28 December 2006 (28.12.2006),<br>paragraphs [0001], [0009] to [0034]; fig. 4<br>(Family: none) | 6-11 |

☐  Further documents are listed in the continuation of Box C.        ☐  See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>17 January, 2014 (17.01.14) | Date of mailing of the international search report<br>28 January, 2014 (28.01.14) |
| Name and mailing address of the ISA/<br>Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012051875 A
- JP 5093545 B
- JP 2005325101 A
- JP 3809462 B